# FASCICULE DE BREVET EUROPEEN

(11) **EP 1 827 511 B1**
(45) Date de publication et mention de la délivrance du brevet: **16.09.2009**
(21) Numéro de dépôt: 05824697.6
(22) Date de dépôt: 12.12.2005
(51) Int. Cl.: A61L 2/10

(54) **DECONTAMINATION LASER DE LA SURFACE D'UNE PIECE PROFILEE**
LASERDEKONTAMINIERUNG DER OBERFLÄCHE EINES FORMTEILS
LASER DECONTAMINATION OF THE SURFACE OF A SHAPED COMPONENT

(30) Priorité: 14.12.2004 FR 0452967
(43) Date de publication de la demande: 05.09.2007
(73) Titulaire: Areva NC, 75009 Paris (FR)
(72) Inventeur: TURCO, Bernard, F-30200 ORSAN (FR); FISCHER, Marc, F-26200 MONTELIMAR (FR)
(74) Mandataire: Poulin, Gérard
(86) Numéro de dépôt international: PCT/FR2005/051071
(87) Numéro de publication internationale: WO 2006/064156

(56) Documents cités:
- EP-A- 0 507 641
- EP-A- 0 520 847
- EP-A- 0 642 846
- FR-A- 2 774 801

## Description

### DOMAINE TECHNIQUE

L'invention concerne la décontamination (en particulier la décontamination radioactive) de la surface d'une pièce profilée au moyen de faisceaux laser.

Par pièce profilée, on entend une pièce de longueur relativement grande par rapport à sa section transversale, cette section restante constante. Il peut s'agir d'un produit métallurgique obtenu par exemple par laminage à chaud ou étirage à froid. Il peut s'agir aussi (et la présente invention portera plus précisément sur ce cas) de crayons de combustible nucléaire.

Plus précisément, l'invention se rapporte au retrait de matière de la surface d'une pièce profilée, consistant à soumettre la surface de la pièce à l'impact d'un faisceau laser pulsé, émettant dans l'ultraviolet, et à aspirer simultanément, au travers d'un filtre, la matière retirée de la surface de la pièce profilée.

Cependant, l'invention peut s'appliquer à tous les domaines de l'industrie dans lesquels il est nécessaire de retirer une couche de matière de la surface d'une telle pièce.

### ÉTAT DE LA TECHNIQUE ANTÉRIEURE

Un crayon (ou aiguille) de combustible nucléaire est constitué par une colonne de matériau fissile contenu dans une gaine. Cette gaine est généralement un tube métallique pourvu de bouchons soudés à ses extrémités. Le matériau fissile est par exemple introduit dans la gaine sous forme de pastilles.

L'introduction des pastilles dans leur gaine présente un risque potentiel de contamination de la surface externe du crayon, en particulier de l'extrémité du crayon accostée au "nez" d'engainage.

Une décontamination des crayons s'avère nécessaire pour les raisons suivantes. Les spécifications techniques exigent que la contamination résiduelle des crayons soit inférieure à un certain seuil : 0,4 Bq/dm² en contamination labile et 83 Bq/dm² en contamination fixée. Ces seuils sont justifiés par la manipulation des crayons dans l'usine, hors confinement.

La contamination présente sur les crayons est constituée de particules submicroniques provenant du matériau constitutif des pastilles de combustible. Dans le cas où le matériau fissile est constitué de pastilles MOX (Mixed OXide), ces particules sont en UO₂, en PuO₂ et en leur mélange. La gaine des crayons doit posséder une bonne tenue mécanique et la conserver jusqu'à la fin de la vie du combustible. Cette caractéristique est notamment mise en évidence par la conservation de l'aspect d'origine de la gaine : blanc, brillant et uniforme. Elle doit notamment rester ductile pour que les déformations répétées, dues aux variations de température et de pression dans les réacteurs, n'entraînent pas de fissuration. On peut avantageusement les réaliser en un alliage de zirconium que l'on appelle le Zircaloy^{®}.

Les quelques projets de nettoyage au laser dans le cadre de la décontamination nucléaire, publiés en presse publique, ont été réalisés aux Etats-Unis et en France. Au début des années 1980, le département américain de l'énergie (DOÉ, US Department Of Energy) proposa d'utiliser les lasers de puissance pour décontaminer les installations nucléaires. Les principaux résultats publiés ont été obtenus entre 1992 et 1996, par une équipe du laboratoire d'Ames dans le cadre du projet « Ames Laser Decontamination Project ». Ils utilisaient à la fois un laser excimère KrF de 100 W (248 nm, 27 ns) et un laser Nd : YAG Q-switch (1064 nm, 100 ns) pour étudier le déplacement de l'oxyde radioactif sur des surfaces métalliques (aluminium, acier, cuivre, fil). L'efficacité de ce procédé d'ablation s'est avérée plus grande avec un plus grand potentiel d'ionisation du gaz ambiant. Des objectifs cylindriques, focalisant le faisceau, ont été utilisés afin de bien couvrir la surface et réduire la redéposition de particules. Ces expériences ont été réalisées avec de faux contaminants, des échantillons radioactifs, et des équipements de grandes dimensions provenant d'installations nucléaires. L'efficacité de la décontamination était en général, suffisante pour ces applications spécifiques. On a proposé le laser Nd : YAG pour développer un prototype puisque sa longueur d'onde (1064 nm) peut être transmise efficacement par des fibres optiques classiques.

D'autres projets se sont axés sur la décontamination du béton. Dans ce cas, les polluants se diffusent en profondeur et la décontamination peut être obtenue par ablation d'une épaisse couche de béton (plusieurs millimètres). Pour ces expériences, des lasers de puissance infrarouge tel que le CO₂ ou le Nd : YAG, sont utilisés. Dans ce but, un prototype de laser de nettoyage à base de Nd : YAG, a alors été conçu au laboratoire « The Argone National Laboratory » et il a probablement été testé pour le déclassement d'un réacteur nucléaire. Récemment, un laser chimique dénommé COIL (pour « Chemical Oxygen Iodine Laser), initialement conçu comme une arme laser militaire, fonctionnant à plus de 1 kW, a été proposé pour le démantèlement d'installations nucléaires.

Depuis 1999, la R et D française suit la même voie. Des expériences ont été réalisées avec des lasers Nd : YAG et excimère. Le document EP-A-0 520 847 divulgue un outil à base de laser pour la décontamination d'un générateur de vapeur provenant d'installations nucléaires. Le document FR-A-2 774 801 divulgue un procédé et une installation de décontamination de crayons de combustible nucléaire au moyen d'un faisceau laser. Les résultats des techniques exposées par ces documents n'ont pas été divulgués.

Le prototype LEXDIN a été conçu et testé par le Commissariat à l'Energie Atomique dans le cas de la décontamination d'une chambre en plexiglas (voir J. R. COSTES et al., « Decontamination by ultraviolet laser : the LEXDIN Prototype », CEA, 1996). Ce prototype utilise un laser XeCl et des miroirs pour le transport du faisceau laser.

Une étude comparative a été réalisée entre le laser Nd : YAG et le laser excimère KrF pour le nettoyage à sec et à chaud d'échantillons contaminés (acier, Inconel) provenant d'un générateur de vapeur. Sous l'atmosphère de l'air, il y avait une faible influence de la longueur d'onde du laser. Un film liquide confinant le plasma permettrait d'augmenter l'efficacité du nettoyage (voir le document FR-A-2 700 882). Comparé au nettoyage à sec, le facteur de décontamination était 30 fois plus grand avec un film d'eau, 85 fois plus grand avec un film d'acide nitrique 0,5 molaire et 650 fois plus grand avec film d'acide nitrique 5 molaire.

D'autres projets de décontamination au moyen d'un faisceau laser ont été entrepris mettant en oeuvre un laser excimère XeCl, un laser Nid : YAG (6 ns) et un laser xénon à lampes flash (200 ms). Cependant, il apparaît qu'aucun système de décontamination par laser n'a été exploité à l'intérieur d'une installation nucléaire pour le nettoyage et le démantèlement.

Le document EP-A -0 507 641 divulgue un procédé de travail au laser sur une surface contenue dans une zone contaminée d'une installation nucléaire. Un faisceau laser pulsé est émis hors de la zone contaminée et est transporté jusqu'à un emplacement voisin de ladite surface. Le faisceau est amplifié puis envoyé sur la surface à traiter, éventuellement par l'intermédiaire d'un miroir de renvoi.

Le document EP-A-0 642 846 divulgue un procédé selon le préambule de la revendication 1.

### EXPOSÉ DE L'INVENTION

La présente invention propose un procédé de décontamination laser et un dispositif mettant en oeuvre ce procédé et exploitable à l'intérieur d'une installation nucléaire.

Un premier objet de l'invention consiste en un procédé de décontamination laser de la surface d'une pièce profilée, consistant à :
- soumettre successivement des parties de la surface de la pièce profilée à l'impact de faisceaux laser pulsés de longueurs d'onde situées dans l'ultraviolet et permettant d'ablater la couche superficielle de la pièce profilée sous forme de particules, les faisceau laser étant répartis de façon à couvrir simultanément chaque partie traitée ;
- récupérer par aspiration lesdites particules;
   caractérisé en ce que chaque partie traitée comprend le pourtour de de pièce profilée.

Avantageusement, lesdits faisceaux laser sont obtenus par division d'un faisceau laser principal et sont d'égale énergie. Avantageusement aussi, lesdits faisceaux laser divisés parcourent chacun un trajet de longueur égale depuis leur division avec le faisceau laser principal

De préférence, la récupération desdites particules est effectuée en filtrant les particules.

Un deuxième objet de l'invention consiste en un dispositif de décontamination laser de la surface d'une pièce profilée, comprenant :
- une chambre de traitement pourvue d'un trou de passage de ladite pièce profilée permettant d'amener successivement des parties de la surface de la pièce profilée dans une zone de traitement, la chambre de traitement comprenant en outre des voies optiques permettant le passage de faisceaux laser pulsés de longueurs d'onde situées dans l'ultraviolet et permettant d'ablater la couche superficielle de la pièce profilée sous forme de particules, les voies optiques étant réparties de façon à couvrir simultanément chaque partie traitée de la surface de la pièce profilée, la chambre de traitement comprenant également des moyens d'aspiration pour récupérer lesdites particules ;
- des moyens pour véhiculer lesdits faisceaux laser jusqu'à la chambre de traitement ; caractérisé en ce que le pourtour de la pièce profilée.

Avantageusement, les moyens d'aspiration comprennent des moyens d'introduction d'un flux de gaz situés de façon que le gaz transite par les voies optiques avant de parvenir à la zone de traitement pour être ensuite dirigé vers une chambre de collecte desdites particules.

Avantageusement, la chambre de collecte comprend des moyens de filtration desdites particules. Les voies optiques peuvent comprendre des moyens de retenue desdites particules destinés à retenir des particules ablatées qui pourraient circuler à contresens du flux de gaz.

Avantageusement, les voies otiques sont des conduits pourvus d'hublots d'étanchéité vers l'extérieur du dispositif et permettant le passage des faisceaux laser.

Avantageusement, les moyens pour véhiculer lesdits faisceaux laser comprennent des moyens de division d'un faisceau laser principal pour obtenir lesdits faisceaux laser d'égale énergie. Ces moyens de division du faisceau laser principal peuvent comprendre des faisceaux de fibres otiques. Ils peuvent comprendre des miroirs semi-réfléchissants et des miroirs à réflexion totale. De préférence, les moyens de division du faisceau laser principal sont des moyens fournissant des faisceaux laser parcourant des trajets égaux.

### BRÈVE DESCRIPTION DES DESSINS

L'invention sera mieux comprise et d'autres avantages et particularités apparaîtront à la lecture de la description qui va suivre, donnée à titre d'exemple non limitatif, accompagnée des dessins annexés parmi lesquels :
- la figure 1A est une vue en coupe transversale de la chambre de traitement d'un dispositif de décontamination d'une pièce profilée, selon la présente invention ;
- la figure 1B est une vue selon le plan BB de la figure 1A ;
- la figure 2 est un premier schéma de principe de-la division d'un faisceau laser principal en quatre faisceaux laser de décontamination et pouvant être mis en oeuvre dans le dispositif de la présente invention ;
- la figure 3 est un deuxième schéma de principe de la division d'un faisceau laser principal en quatre faisceaux laser de décontamination et pouvant être mis en oeuvre dans le dispositif de la présente invention ;
- la figure 4 est un troisième schéma de principe de la division d'un faisceau laser principal en quatre faisceaux laser de décontamination et pouvant être mis en oeuvre dans le dispositif de la présente invention ;
- la figure 5 est un quatrième schéma de principe de la division d'un faisceau laser principal en quatre faisceaux laser de décontamination et pouvant être mis en oeuvre dans le dispositif de la présente invention.

### EXPOSÉ DÉTAILLÉ DE MODES DE RÉALISATION PARTICULIERS

Lors de l'irradiation d'une surface par un laser impulsionnel, une partie de l'énergie laser est absorbée par le matériau, et cela crée un gradient de température dans l'épaisseur du matériau qui dépend de son absorptivité à la longueur d'onde du laser. Trois processus sont principalement à l'origine du phénomène d'ablation : un mécanisme thermique, un mécanisme mécanique et un mécanisme photochimique. Ces mécanismes sont étroitement liés, mais en fonction de la nature du substrat, de la longueur d'onde et de la durée d'impulsion du laser, l'un d'eux devient prédominant dans le processus d'ablation.

L'absorptivité des matériaux métalliques est d'autant plus importante que la longueur d'onde du laser est courte. Les photons très énergétiques (4 à 8 eV) des lasers à excimères sont absorbés par une faible épaisseur du matériau (quelques nanomètres) et la longueur de diffusion thermique est typiquement de l'ordre du micromètre. Ils transfèrent leur énergie aux électrons du matériau qui en se désexcitant vont provoquer l'échauffement de la matière irradiée. Lorsque la température du volume qui a absorbé l'énergie laser est supérieure à la température de vaporisation du matériau, le processus d'ablation est initié. Ce processus d'ablation d'origine thermique se caractérise par la formation d'un plasma composé par des électrons et des espèces neutres et ionisées issues du substrat irradié et du gaz ambiant. Ce plasma se propage perpendiculairement à la surface du matériau. La forte absorptivité du substrat dans l'UV permet de n'élever la température que dans un faible volume, et donc de n'ablater qu'une fine couche du matériau tout en limitant les effets thermiques plus en profondeur. Les courtes durées d'impulsion des lasers à excimères, entre 10 et 30 ns, limitent encore la diffusion de la chaleur, et donc les effets thermiques à l'intérieur du volume non ablaté.

Pour les faibles fluences, la température atteinte à la surface n'est pas suffisante pour provoquer la vaporisation du matériau, mais l'absorption de l'énergie par la surface, et/ou les composés (particules, couches) qui y sont déposés, induit une augmentation rapide de leur température. Ceci va provoquer une expansion brusque des volumes chauffés et, si l'accélération qui est alors transmise aux composés déposés sur la surface est suffisante, ils peuvent être éjectés de celle-ci. Ce processus d'ablation d'origine mécanique ne modifie pas la structure et la morphologie du substrat. Il est prédominant lors du nettoyage de micro-particules par laser. Il peut être mis en oeuvre par voie sèche en irradiant directement la surface par le faisceau laser ou par voie humide en déposant un film liquide sur la surface avant l'irradiation, et dans ce cas c'est l'ablation du film qui va provoquer l'éjection des particules. Il est à l'origine de l'enlèvement des particules d'oxyde. Il est efficace car les matériaux (substrat et particules) absorbent fortement le rayonnement laser UV.

Les processus induits par l'irradiation d'une surface par un faisceau ultraviolet peuvent également être d'origine photochimique lorsque l'énergie des photons (4 à 8 eV) est supérieure aux énergies de liaison des composés irradiés. Ce processus d'ablation d'origine photochimique se produit en général pour des substrats tels que les polymères. Les principales applications sont le décapage de peinture et la gravure de matériaux polymères.

L'invention concerne un procédé de retrait de matière de la surface d'une pièce profilée, consistant à soumettre la surface de la pièce profilée à l'impact d'un faisceau laser pulsé émettant dans l'ultraviolet et à aspirer simultanément au travers d'un filtre la matière retirée de la surface de la pièce profilée. C'est un mécanisme mécanique qui est mis en oeuvre par l'invention.

Un dispositif de décontamination de pièces profilées, conformément à la présente invention comprend une chambre de traitement maintenue en dépression et un système de mise en forme d'un faisceau laser.

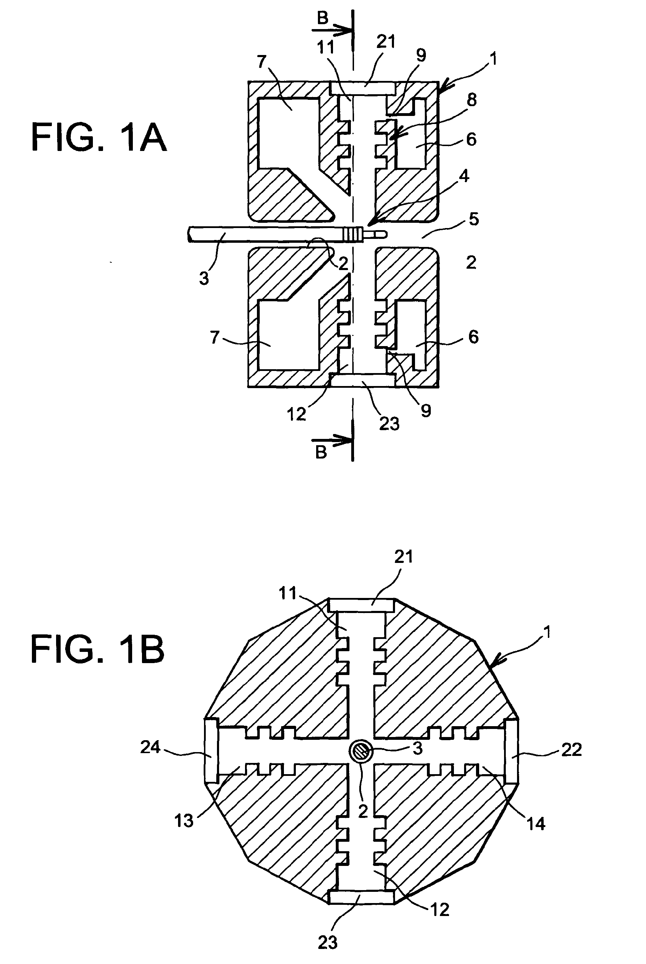

La chambre de traitement 1 est représentée aux figures 1A et 1B. Elle est pourvue d'un trou central 2 permettant l'introduction d'une pièce profilée 3 à traiter. Le traitement de la pièce profilée 3 est réalisé dans la zone référencée 4 sur la figure 1A. La zone 4 correspond à la jonction de quatre voies optiques 11, 12, 13 et 14 d'égales longueurs et disposées selon deux axes orthogonaux. Les voies optiques 11, 12, 13 et 14 sont obturées par des hublots respectivement 21, 22, 23 et 24 qui assurent l'étanchéité avec l'extérieur de la chambre de traitement 1 et qui permettent le passage des faisceaux laser nécessaires à la décontamination.

La pièce profilée à décontaminer est introduite dans la chambre de traitement 1 par le trou central 2 muni d'une cage à billes non représentée, afin d'assurer le parfait centrage de la pièce. La pièce profilée 3 avance à travers la chambre à une vitesse donnée. Les paramètres vitesse d'avancée de la pièce et fréquence des faisceaux laser permettent d'obtenir le nombre d'impulsions laser nécessaire à la décontamination.

Une ou plusieurs pompes à vide permettent de créer une dépression à l'intérieur de la chambre de traitement, ce qui permet d'aspirer les contaminants grâce à un ensemble de filtres THE (filtres à très haute efficacité).

L'air pénètre dans la chambre de décontamination de deux façons : par l'orifice 5 de sortie des pièces profilées et par une chambre annulaire 6 situées près des hublots et communiquant avec les voies optiques par des canaux 9. Cet arrangement permet également d'éviter un redépôt des contaminants sur les hublots, ce qui pourrait affecter l'efficacité de la décontamination. Sous l'impact des faisceaux laser sur la surface de la pièce profilée 3, des particules sont aspirées du fait de la dépression existant à l'intérieur de la chambre de décontamination. Ces particules sont dirigées, selon un angle de 45° par rapport à l'axe du faisceau laser incident, dans une chambre de collecte annulaire 7 pour être ensuite capturées par les filtres THE. Pour être efficace, l'aspiration se fait au plus près de la zone de traitement 4. Sur les trajets des faisceaux laser, c'est-à-dire dans les voies optiques 11, 12, 13 et 14, des créneaux 8 sont agencés pour empêcher les particules arrachées de venir se déposer sur la surface intérieure des hublots 21, 22, 23 et 24.

Les faisceaux laser de traitement sont avantageusement issus de la division d'un faisceau laser principal. La distance parcourue par chacun des faisceaux laser incidents est identique depuis le point de division du faisceau laser principal jusqu'à la zone de traitement, ce qui assure une parfaite homogénéité du traitement. Plusieurs faisceaux laser permettent d'illuminer simultanément le pourtour de la pièce profilée. Dans l'exemple décrit ici, les faisceaux divisés sont au nombre de quatre mais le nombre de faisceaux divisés peut être différent.

Le faisceau laser principal peut être transporté par un faisceau de fibres optiques. Les faisceaux laser divisés ou secondaires sont alors créés par la division du faisceau de fibres optiques principal.

Le faisceau laser principal peut être transportée en réflexion sur des miroirs. Les faisceaux laser secondaires sont créés par la division du faisceau laser principal du moyen de miroirs et de lames séparatrices.

Le circuit d'aspiration des particules arrachées est défini avec des sections de passage assez faibles pour maintenir des vitesses d'écoulement (gaz + particules) élevées et ainsi limiter les dépôts de particules qui peuvent s'accrocher aux surfaces par des effets mécaniques ou par des forces de Van Der Waals dont les potentiels peuvent atteindre 2 à 5 eV suivant les matériaux.

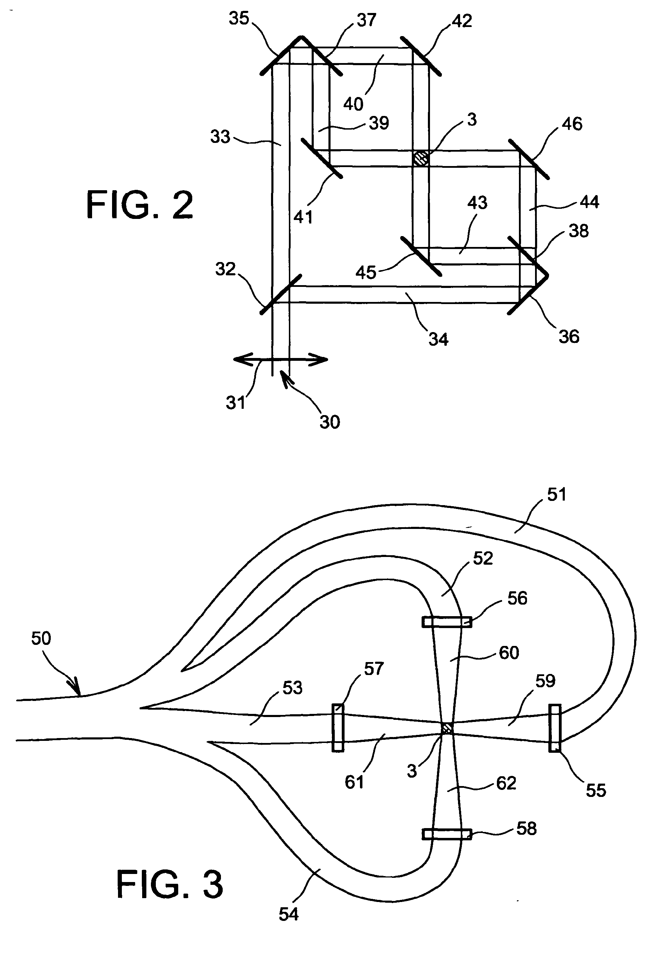

Les figures 2 à 5 sont différents schémas de principe de la division d'un faisceau laser principal en quatre faisceaux de décontamination et pouvant être mis en oeuvre dans le dispositif de la présente invention.

Sur le schéma de la figure 2, un faisceau laser principal 30, mis en forme par une lentille 31, est envoyé sur un miroir 32 à 50% de réflexion. Le miroir 32 divise le faisceau laser principal en deux faisceaux 33 et 34 qui sont dirigés vers des miroirs à 100% de réflexion, respectivement 35 et 36. Les faisceaux 33 et 34 sont alors réfléchis vers des miroirs à 50% de réflexion, respectivement 37 et 38. Le faisceau 33 est alors divisé en deux faisceaux secondaires 39 et 40 qui sont renvoyés, respectivement par les miroirs à 100% de réflexion 41 et 42, vers la surface de la pièce profilée 3. Le faisceau 34 est lui aussi divisé en deux faisceaux secondaires 43 et 44 qui sont renvoyés, respectivement par les miroirs à 100% de réflexion 45 et 46, vers la surface de la pièce profilée 3. Les quatre faisceaux secondaires 39, 40, 43 et 44 sont d'égale énergie et ont parcouru la même distance jusqu'à la pièce profilée 3.

Sur le schéma de la figure 3, un faisceau laser principal est véhiculé par un faisceau de fibres optiques principal 50. Le faisceau de fibres optiques principal 50 est séparé en quatre faisceaux de fibres secondaires 51, 52, 53 et 54 d'égales longueurs (contrairement à ce que montre le schéma de la figure 3 qui est un schéma de principe). Les faisceaux de fibres secondaires 51, 52, 53 et 54 sont amenés en face d'optiques de remise en forme du faisceau, respectivement 55, 56, 57 et 58 qui fournissent des faisceaux respectivement 59, 60, 61 et 62 d'égale énergie et incidents à la surface de la pièce profilée 3.

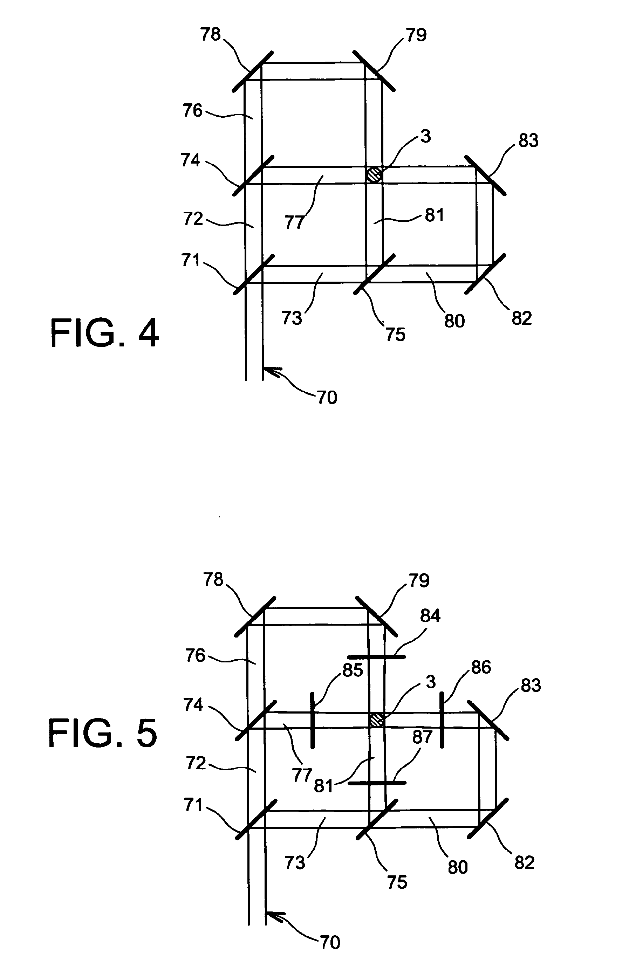

Sur le schéma de la figure 4, un faisceau laser principal 70 est envoyé sur un miroir 71 à 50% de réflexion. Le miroir 71 divise le faisceau laser principal en deux faisceaux 72 et 73 qui sont dirigés vers d'autres miroirs à 50% de réflexion, respectivement 74 et 75. Le faisceau 72 est alors divisé en deux faisceaux secondaires 76 et 77 qui sont dirigés vers la pièce profilée 3, directement pour le faisceau secondaire 77 et par l'intermédiaire de deux miroirs à 100% de réflexion 78 et 79 pour le faisceau secondaire 76. Le faisceau 73 est lui aussi divisé en deux faisceaux secondaires 80 et 81 qui sont dirigés vers la pièce profilée 3, directement pour le faisceau secondaire 81 et par l'intermédiaire de deux miroirs à 100% de réflexion 82 et 83 pour le faisceau secondaire 80. La pièce profilée 3 est alors soumise à l'impact de quatre faisceaux parallèles et d'égales énergies.

Sur le schéma de la figure 5, on retrouve la même structure que le schéma de la figure 4. Sur le schéma de la figure 5, des optiques de focalisation 84, 85, 86 et 87 ont été ajoutées sur le trajet des faisceaux secondaires, respectivement 76, 77, 80 et 81, incidents à la surface de la pièce profilée 3. La pièce profilée 3 est alors soumise à l'impact de quatre faisceaux convergents et d'égales énergies.

## Revendications

1. Procédé de décontamination laser de la surface d'une pièce profilée (3), consistant a :
- soumettre successivement des parties de la surface de la pièce profilée (3) à l'impact de faisceaux laser pulsés (39, 40, 43, 44) de longueurs d'onde situées dans l'ultraviolet et permettant d'ablater la couche superficielle de la pièce profilée sous forme de particules, les faisceaux laser étant répartis de façon à couvrir simultanément chaque partie traitée ;
- récupérer par aspiration lesdites particules;
**caractérisé en ce que** chaque partie traitée comprend le pourtour de la pièce profilée.

2. Procédé selon la revendication 1,
**caractérisé en ce que** lesdits faisceaux laser (39, 40, 43, 44) sont obtenus par division d'un faisceau laser principal (30) et sont d'égale énergie.

3. Procédé selon la revendication 2,
**caractérisé en que** lesdits faisceaux laser divisés parcourent chacun un trajet de longueur égale depuis leur division avec le faisceau laser principal.

4. Procédé selon l'une quelconque des
revendications 1 à 3, **caractérisé en ce que** la récupération desdites particules est effectuée en filtrant les particules.

5. Dispositif de décontamination laser de la surface d'une pièce profilée, comprenant :
- une chambre de traitement (1) pourvue d'un trou de passage (2) de ladite pièce profilée (3) permettant d'amener successivement des parties de la surface de la pièce profilée dans une zone de traitement (4), la chambre de traitement (1) comprenant en outre des voies optiques (11, 12, 13, 14) permettant le passage de faisceaux laser pulsés de longueurs d'onde situées dans l'ultraviolet et permettant d'ablater la couche superficielle de la pièce profilée sous forme de particules, les voies optiques (11, 12, 13, 14) étant réparties de façon à couvrir simultanément chaque partie traitée de la surface de la pièce profilée (3), la chambre de traitement (1) comprenant également des moyens d'aspiration pour récupérer lesdites particules ;
- des moyens pour véhiculer lesdits faisceaux laser jusqu'à la chambre de traitement;
**caractérisé en ce que** les voies optiques couvrent, en outre, le pourtour de la pièce profilée.

6. Dispositif selon la revendication 5,
**caractérisé en ce que** les moyens d'aspiration comprennent des moyens d'introduction d'un flux de gaz (6, 9) situés de façon que le gaz transite par les voies optiques (11, 12, 13, 14) avant de parvenir à la zone de traitement pour être ensuite dirigé vers une chambre de collecte (7) desdites particules.

7. Dispositif selon la revendication 5,
**caractérisé en ce que** la chambre de collecte (7) comprend des moyens de filtration desdites particules.

8. Dispositif selon l'une des revendications 6 ou 7, **caractérisé en ce que** les voies otiques (11, 12, 13, 14) comprennent des moyens de retenue (8) desdites particules destinés à retenir des particules ablatées qui pourraient circuler à contresens du flux de gaz.

9. Dispositif selon l'une quelconque des revendications 5 à 8, **caractérisé en ce que** les voies otiques (11, 12, 13, 14) sont des conduits pourvus d'hublots d'étanchéité (21, 22, 23, 24) vers l'extérieur du dispositif (1) et permettant le passage des faisceaux laser.

10. Dispositif selon l'une quelconque des revendications 5 à 9, **caractérisé en ce que** les moyens pour véhiculer lesdits faisceaux laser comprennent des moyens de division d'un faisceau laser principal pour obtenir lesdits faisceaux laser d'égale énergie.

11. Dispositif selon la revendication 10, **caractérisé en ce que** les moyens de division du faisceau laser principal comprennent des faisceaux de fibres optiques (50, 51, 52, 53, 54).

12. Dispositif selon la revendication 10, **caractérisé en ce que** les moyens de division du faisceau laser principal comprennent des miroirs semi-réfléchissants (32, 37, 38) et des miroirs à réflexion totale (35, 36, 41, 42, 45, 46).

13. Dispositif selon l'une quelconque des revendications 10 à 12, **caractérisé en ce que** les moyens de division du faisceau laser principal sont des moyens fournissant des faisceaux laser parcourant des trajets égaux.

## Claims

1. A method for laser decontamination of the surface of a profiled part (3), consisting in :
- successively submitting portions of the surface of the profiled part (3) to the impact of pulsed laser beams (39, 40, 43, 44) with wavelengths located in the ultraviolet and with which the surface layer of the profiled part may be ablated as particles, the laser beams being distributed so as to simultaneously cover each treated portion;
- recovering said particles by suction ;
**characterized in that** each treated portion comprises the periphery of the profiled part.

2. The method according to claim 1,
**characterized in that** said laser beams (39, 40, 43, 44) are obtained by dividing a main laser beam (30) and are of equal energy.

3. The method according to claim 2,
**characterized in that** said divided laser beams each travel over a trajectory of equal length from their division with the main laser beam.

4. The method according to any of claims 1 to 3, **characterized in that** the recovery of said particles is carried out by filtering the particles.

5. A device for laser decontamination of the surface of a profiled part, comprising :
- a treatment chamber (1) provided with a passage hole (2) for said profiled part (3) with which portions of the surface of the profiled part may be successively brought into a treatment area (4), the treatment chamber (1) further comprising optical paths (11, 12, 13, 14) providing the passage for pulsed laser beams with wavelengths located in the ultraviolet and with which the surface layer of the profiled part may be ablated as particles, the optical paths (11, 12, 13, 14) being distributed so as to simultaneously cover each treated portion of the surface of the profiled part (3), the treatment chamber (1) also comprising suction means for recovering said particles;
- means for conveying said laser beams up to the treatment chamber ;
**characterized in that** the optical paths further cover the periphery of the profiled part.

6. The device according to claim 5,
**characterized in that** the suction means comprise means for introducing a gas jet (6, 9) located so that the gas transits via the optical paths (11, 12, 13, 14) before reaching the treatment area where it is subsequently directed towards a chamber (7) for collecting said particles.

7. The device according to claim 5,
**characterized in that** the collection chamber (7) comprises means for filtering said particles.

8. The device according to any of claims 6 or 7, **characterized in that** the optical paths (11, 12, 13, i4) comprise means (8) for retaining said particles, intended to retain ablated particles which may flow in the counter-direction to the gas jet.

9. The device according to any of claims 5 to 8, **characterized in that** the optical paths (11, 12, 13, 14) are conduits provided with sealing windows (21, 22, 23, 24) towards the outside of the device (1) and providing the passage for the laser beams.

10. The device according to any of claims 5 to 9, **characterized in that** the means for transporting said laser beams comprise means for dividing a main laser beam in order to obtain said laser beams with equal energy.

11. The device according to claim 10,
**characterized in that** the means for dividing the main laser beam comprise bundles of optical fibers (50, 51, 52, 53, 54).

12. The device according to claim 10,
**characterized in that** the means for dividing the main laser beams comprise semi-reflecting mirrors (32, 37, 38) and total reflection mirrors (35, 36, 41, 42, 45, 46).

13. The device according to any of claims 10 to 12, **characterized in that** the means for dividing the main laser beam are means providing laser beams traveling over equal trajectories.

## Patentansprüche

1. Verfahren zum Laserdekontaminieren der Oberfläche eines Profilteils (3), das aus Folgendem besteht:
- allmähliches Aussetzen der Teile der Oberfläche des Profilteils (3) mit dem Aufprallen gepulster Laserstrahlen (39, 40, 43, 44) mit Wellenlängen, die im Ultraviolettbereich liegen und es erlauben, die oberflächliche Schicht des Profilteils in Form von Teilchen abzutragen, wobei die Laserstrahlen derart verteilt sind, dass jeder behandelte Teil gleichzeitig bedeckt ist;
- Rückgewinnen der Teilchen durch Aufsaugen;
**dadurch gekennzeichnet, dass** jeder behandelte Teil den Umfang des Profilteils umfasst.

2. Verfahren nach Anspruch 1, **dadurch gekennzeichnet, dass** die Laserstrahlen (39, 40, 43, 44) durch Teilen eines Hauptlaserstrahls 30 erzielt werden und gleiche Energie aufweisen.

3. Verfahren nach Anspruch 2, **dadurch gekennzeichnet, dass** die geteilten Laserstrahlen jeweils einen Weg mit gleicher Länge ab ihrer Teilung von dem Hauptlaserstrahl zurücklegen.

4. Verfahren nach einem der Ansprüche 1 bis 3, **dadurch gekennzeichnet, dass** das Rückgewinnen der Teilchen durch Filtern der Teilchen erfolgt.

5. Laserdekontaminationsvorrichtung der Oberfläche eines Profilteils mit:
- einer Behandlungskammer (1), die mit einer Durchgangsbohrung (2) des Profilteils (3) versehen ist, die es erlaubt, allmählich Teile der Oberfläche des Profilteils in eine Behandlungszone (4) zu bringen, wobei die Behandlungskammer (1) feiner optische Kanäle (11, 12, 13, 14) aufweist, die das Durchgehen gepulster Laserstrahlen mit Wellenlänge im Ultraviolettbereich erlaubten und das Abtragen der oberflächlichen Schicht des Profilteils in Form von Teilchen erlauben, wobei die optischen Kanäle (11, 12, 13, 14) derart verteilt sind, dass sie gleichzeitig jeden behandelten Teil der Oberfläche des Profilteile (3) decken, wobei die Behandlungskammer (1) auch Mittel zum Absaugen aufweist, um die Teilchen zurückzugewinnen;
- Mittel zum Transportieren der Laserstrahlen bis zu der Behandlungskammer;
**dadurch gekennzeichnet, dass** die optischen Kanäle ferner den Umfang des Profilteils abdecken.

6. Vorrichtung nach Anspruch 5, **dadurch gekennzeichnet, dass** die Ansaugmittel Mittel zum Einführen eines Gasflusses (6, 9) aufweise, die derart angeordnet sind, dass das Gas durch die optischen Kanäle (11, 12, 13, 14) durchgeht, bevor es zu der Behandlungszone gelangt, um danach zu einer Sammelkammer (7) der Teilchen gelenkt zu werden.

7. Vorrichtung nach Anspruch 5, **dadurch gekennzeichnet, dass** die Sammelkammer (7) Filtermittel der Teilchen aufweist.

8. Vorrichtung nach einem der Ansprüche 6 oder 7, **dadurch gekennzeichnet, dass** die optischen Kanäle (11, 12, 13, 14) Rückhaltemittel (8) der Teilchen aufweisen, die dazu bestimmt sind, abgetragene Teilchen, die gegen die Gasströmungsrichtung zirkulieren könnten, zurückzuhalten.

9. Vorrichtung nach einem der Ansprüche 5 bis 8, **dadurch gekennzeichnet, dass** die optischen Kanäle (11, 12, 13, 14) Leitungen sind, die mit Abdichtbullaugen (21, 22, 23, 24) zur Außenseite der Vorrichtung (1), die das Durchgehen der Laserstrahlen erlauben, versehen sind

10. Vorrichtung nach einem der Ansprüche 5 bis 9, **dadurch gekennzeichnet, dass** die Mittel zum Transportieren der Laserstrahlen Mittel zum Teilen eines Hauptlaserstrahls aufweisen, um die Laserstrahlen mit gleicher Energie zu erzielen.

11. Vorrichtung nach Anspruch 10, **dadurch gekennzeichnet, dass** die Mittel zum Teilen des Hauptlaserstrahls Optikfaserbündel (50, 51, 52, 53, 54) aufweisen.

12. Vorrichtung nach Anspruch 10, **dadurch gekennzeichnet, dass** die Mittel zum Teilen des Hauptlaserstrahls halb reflektierende Spiegel (32, 37, 38) und Spiegel mit kompletter Reflexion (35, 36, 41, 42, 45, 46) aufweisen.

13. Vorrichtung nach einem der Ansprüche 10 bis 12, **dadurch gekennzeichnet, dass** die Mittel zum Teilen des Hauptlaserstrahls Mittel sind, die Laserstrahlen liefern, die gleiche Wege zurücklegen.
